# FASCICULE DE BREVET EUROPEEN

(11) **EP 1 792 638 B1**
(45) Date de publication et mention de la délivrance du brevet: **03.06.2009**
(21) Numéro de dépôt: 06291808.1
(22) Date de dépôt: 23.11.2006
(51) Int. Cl.: A61M 25/01, A61B 17/00, A61B 19/00

(54) **Enrouleur/dérouleur à cathéters et système d'artériographie muni d'un tel enrouleur/dérouleur**
Auf/Abroller für Katheter und Arteriographiesystem mit einem solchen Auf/Abroller
Winder/unwinder for catheters and system of arteriography comprising such a winder/unwinder

(30) Priorité: 30.11.2005 FR 0512166
(43) Date de publication de la demande: 06.06.2007
(73) Titulaire: Bencteux, Philippe, 76230 Bois-Guillaume (FR)
(72) Inventeur: Bencteux, Philippe, 76230 Bois-Guillaume (FR)
(74) Mandataire: Cousin, Geoffroy

(56) Documents cités:
- EP-A- 1 442 720
- WO-A-00/33723
- WO-A-20/05000105
- US-A1- 2002 038 116

## Description

La présente invention est relative aux enrouleurs/dérouleurs à cathéters et aux systèmes d'artériographie munis de tels enrouleurs/dérouleurs.

L'introduction manuelle par un praticien de cathéters ou guides à l'intérieur du corps d'un patient, par exemple en vue de réaliser une artériographie, n'est pas satisfaisante car celle-ci est généralement réalisée sous rayons X pour permettre au praticien de visualiser le déplacement de l'extrémité du cathéter à l'intérieur du corps du patient au cours de l'introduction. Il en résulte une irradiation du praticien qui, si elle n'est pas plus nocive en soi que l'irradiation du patient, est problématique car le praticien est amené à répéter cette opération sur de nombreux patients, ce qui le conduit à être exposé à une dose de radiations conséquentes.

Par conséquent, on a recherché des moyens d'automatiser et de contrôler à distance cette artériographie par un automate, ce qui permet au praticien de guider à distance l'introduction du cathéter dans le corps du patient.

Le document W02005/000,105 décrit de nombreux exemples de tels automates. Toutefois, dans tous ces modes de réalisation, un problème demeure. En effet, pendant l'introduction, la partie non encore introduite des différents cathéters ou guides doit être maintenue dans un milieu aqueux adapté pour éviter leur contamination par des éléments extérieurs, ce qui n'est rendu possible par aucun des modes de réalisation présentés dans ce document.

La présente invention a notamment pour but de pallier ces inconvénients.

A cet effet, selon l'invention, on prévoit un enrouleur/dérouleur à cathéters comprenant au moins un premier système et un deuxième système,
le premier système comprenant :
- un premier récipient adapté pour recevoir un premier organe tubulaire creux allongé à introduire dans un canal d'un patient et s'étendant entre une première extrémité et une deuxième extrémité le long d'une direction d'élongation du premier organe,
   ledit premier récipient comportant une ouverture formée pour laisser passer ledit premier organe vers l'extérieur du premier système,
- un premier mécanisme d'entraînement adapté pour appliquer au premier organe un mouvement à travers ladite ouverture par rapport audit premier récipient,
   le deuxième système comprenant :

- un deuxième récipient adapté pour recevoir un deuxième organe tubulaire allongé à introduire dans le canal et s'étendant entre une première extrémité et une deuxième extrémité le long d'une direction d'élongation de deuxième organe,
   ledit deuxième récipient comportant une sortie formée pour laisser passer le deuxième organe vers l'extérieur du deuxième système et vers l'intérieur du premier système,
   ledit deuxième récipient étant monté rotatif par rapport au premier récipient autour d'un axe de rotation,
- un deuxième mécanisme d'entraînement adapté pour appliquer au deuxième organe au moins un mouvement le long de ladite direction d'élongation de deuxième organe par rapport audit deuxième récipient,
   ladite sortie comportant un dispositif de fixation au premier organe, adapté pour un déplacement solidaire du premier organe et du deuxième récipient le long de la direction d'élongation de premier organe.

Grâce à ces dispositions, on obtient un enrouleur/dérouleur à cathéters qui se présente sous forme de récipients pouvant contenir à la fois le liquide de conservation et les cathéters en question. De plus, l'ensemble est réalisé de manière compacte et peut être réalisé sous forme de consommables. La réalisation autour d'un axe de rotation unique permet également un arrangement modulaire qui permet d'utiliser facilement deux ou plus cathéters lors d'une opération, ce qui est souvent le cas en pratique, sans avoir à prévoir de nombreuses manipulations du mécanisme pendant l'opération d'introduction.

Dans des modes de réalisation préférés de l'invention, on peut éventuellement avoir recours en outre à l'une et/ou à l'autre des dispositions suivantes :
- le premier récipient présente un fond adapté pour recevoir ledit premier organe allongé, et au moins une première surface de guidage,
   le deuxième récipient présente un fond adapté pour recevoir ledit guide allongé, et au moins une deuxième surface de guidage coopérant avec ladite première surface de guidage du premier récipient pour guider un mouvement relatif des premier et deuxième récipient autour dudit axe de rotation ;
- le premier mécanisme d'entraînement est adapté pour appliquer au premier organe au moins l'un des mouvements suivants par rapport audit premier récipient :
   - une translation le long de ladite direction d'élongation de premier organe,
   - une rotation autour de ladite direction d'élongation de premier organe ;
- le deuxième mécanisme d'entraînement est adapté pour appliquer au deuxième organe par rapport audit deuxième récipient au moins l'un des mouvements suivants :
   - une translation le long de ladite direction d'élongation de deuxième organe, et
   - une rotation autour de ladite direction d'élongation de deuxième organe ;
- un mécanisme de motorisation est adapté pour impartir un mouvement relatif des premier et deuxième récipients autour dudit axe de rotation ;
- l'enrouleur/dérouleur à cathéters comprend en outre, à titre de premier organe, un cathéter allongé s'étendant dans le premier récipient entre une première extrémité et une deuxième extrémité le long d'une direction d'élongation de premier organe, ladite deuxième extrémité étant fixée audit dispositif de fixation, le cathéter allongé coopérant avec ledit premier mécanisme d'entraînement pour que celui-ci lui applique un mouvement par rapport audit premier récipient ;
- l'enrouleur/dérouleur à cathéters comprend en outre, à titre de deuxième organe, un guide allongé s'étendant dans le deuxième récipient entre une première extrémité et une deuxième extrémité le long d'une direction d'élongation de deuxième organe, le guide allongé coopérant avec ledit deuxième mécanisme d'entraînement pour que celui-ci lui applique un mouvement par rapport audit deuxième récipient,
   ladite première extrémité du guide passant à travers ladite sortie à l'intérieur du cathéter ;
- les récipients contiennent un liquide adapté à la conservation d'organes à introduire dans un canal d'un patient ;
- lesdits mécanismes d'entraînement sont réalisés amovibles par rapport à leur système respectif ;
- le deuxième récipient est adapté pour recevoir en outre un troisième organe tubulaire allongé à introduire dans le canal et s'étendant entre une première extrémité et une deuxième extrémité le long d'une direction d'élongation de troisième organe, le deuxième système comprenant un troisième mécanisme d'entraînement adapté pour appliquer au troisième organe au moins un mouvement le long de ladite direction d'élongation de troisième organe par rapport audit deuxième récipient, sans appliquer de mouvement au deuxième organe, le deuxième mécanisme d'entraînement étant adapté pour appliquer au deuxième organe ledit mouvement le long de la direction d'élongation de deuxième organe sans appliquer de mouvement au troisième organe ;
- l'enrouleur/dérouleur à cathéters comprend en outre un troisième système comprenant :
   - un troisième récipient adapté pour recevoir un troisième organe tubulaire allongé à introduire dans le canal et s'étendant entre une première extrémité et une deuxième extrémité le long d'une direction d'élongation de troisième organe,
      ledit troisième récipient comportant une sortie formée pour laisser passer le troisième organe,
      ledit troisième récipient étant monté rotatif par rapport au deuxième récipient autour dudit axe de rotation,
   - un troisième mécanisme d'entraînement adapté pour appliquer au troisième organe au moins un mouvement à travers ladite sortie du troisième récipient le long de ladite direction d'élongation de troisième organe par rapport audit troisième récipient,
      la sortie du troisième récipient comportant un dispositif de fixation au deuxième organe, adapté pour un déplacement solidaire du deuxième organe et du troisième récipient le long de la direction d'élongation de deuxième organe ;
- l'enrouleur/dérouleur à cathéters comprend en outre un troisième système comprenant :
   - un troisième récipient adapté pour recevoir un troisième organe tubulaire allongé à introduire dans le canal et s'étendant entre une première extrémité et une deuxième extrémité le long d'une direction d'élongation de troisième organe,
      ledit troisième récipient comportant une sortie formée pour laisser passer le troisième organe,
      ledit troisième récipient étant monté rotatif par rapport au deuxième récipient autour dudit axe de rotation,
   - un troisième mécanisme d'entraînement adapté pour appliquer au troisième organe au moins un mouvement à travers ladite sortie du troisième récipient le long de ladite direction d'élongation de troisième organe par rapport audit troisième récipient,
      la sortie du troisième récipient comportant un dispositif de fixation au premier organe, adapté pour un déplacement solidaire du premier organe et du troisième récipient le long de la direction d'élongation de premier organe ;
- au moins un mécanisme d'entraînement comprend un dispositif électrique de motorisation comprenant au moins un moteur électrique adapté pour générer, lors du passage d'un courant électrique, un mouvement relatif du récipient et d'un élément à déplacer correspondant choisi parmi lesdits organes ;
- ledit mécanisme d'entraînement comprend un équipage monté mobile par rapport au récipient selon un premier degré de liberté,
   ledit élément à déplacer est monté mobile sur ledit équipage selon un deuxième degré de liberté distinct du premier degré de liberté,
   ledit dispositif électrique de motorisation étant adapté pour générer un mouvement relatif de l'équipage et du récipient selon le premier degré de liberté, afin de générer un mouvement relatif de l'élément à déplacer et du récipient selon ledit premier degré de liberté,
   ledit dispositif électrique de motorisation étant adapté pour générer un mouvement relatif de l'équipage et de l'élément à déplacer selon le deuxième degré de liberté, afin de générer un mouvement relatif de l'élément à déplacer et du récipient selon ledit deuxième degré de liberté ;
- ledit dispositif électrique de motorisation comprend au moins un moteur électrique, lesdits moteurs électriques étant disposés sur le récipient et non sur l'équipage, ledit dispositif électrique de motorisation comprenant au moins un élément de transfert de mouvement relié à un desdits moteurs électriques pour transmettre ledit mouvement relatif de l'élément à déplacer et de l'équipage selon le deuxième degré de liberté depuis ledit moteur électrique ;
- chaque récipient est en forme de cuve.

Selon un autre aspect, l'invention se rapporte à un système d'artériographie comprenant un tel enrouleur/dérouleur à cathéters.

Dans un mode de réalisation, le système d'artériographie comprend en outre une unité centrale adaptée pour commander les mécanismes d'entraînement.

Selon un autre aspect, l'invention se rapporte à une installation comprenant un enrouleur/dérouleur à cathéter comprenant un récipient adapté pour recevoir un organe tubulaire allongé à introduire dans un canal d'un patient et s'étendant entre une première extrémité et une deuxième extrémité le long d'une direction d'élongation,
le récipient comprenant une ouverture formée pour laisser passer ledit organe vers l'extérieur,
l'enrouleur/dérouleur comprenant en outre un mécanisme d'entraînement adapté pour appliquer à l'organe un mouvement à travers ladite ouverture par rapport audit récipient, ledit mécanisme d'entraînement comprenant :
- un équipage monté mobile par rapport au récipient selon un premier degré de liberté, l'équipage portant l'organe,
- un dispositif électrique de motorisation adapté pour générer un mouvement lors du passage d'un courant électrique,
   ledit dispositif électrique de motorisation étant adapté pour générer un mouvement relatif de l'équipage et du récipient selon le premier degré de liberté, afin de générer un mouvement relatif de l'organe et du récipient selon ledit premier degré de liberté,
   l'organe étant monté mobile par rapport à l'équipage selon un deuxième degré de liberté distinct du premier degré de liberté,
   ledit dispositif électrique de motorisation étant adapté pour générer un mouvement relatif de l'équipage et de l'organe selon le deuxième degré de liberté, afin de générer un mouvement relatif de l'organe et du récipient selon ledit deuxième degré de liberté,
   ledit dispositif électrique de motorisation comprenant au moins un moteur électrique, ledit dispositif électrique de motorisation comprenant au moins un élément de transfert de mouvement relié à un desdits moteurs électriques pour transmettre ledit mouvement relatif de l'organe et de l'équipage selon le deuxième degré de liberté depuis ledit moteur électrique,
   l'installation comportant une partie à demeure, comportant lesdits moteurs électriques, et une partie remplaçable utilisée, distincte dudit organe, montée de manière amovible par rapport à la partie à demeure, la partie à demeure et la partie remplaçable formant, dans un état monté, ledit enrouleur/dérouleur, l'installation comportant une pluralité de parties remplaçables de remplacement, identiques à la partie remplaçable utilisée.

Une telle installation permet d'utiliser comme consommables une partie remplaçable, ce qui permet de garantir les conditions d'hygiène requises pour une telle installation, tout en minimisant les coûts, car seule la partie consommable est remplacée entre deux utilisations.

Dans des modes de réalisation préférés de cette installation, on peut en outre avoir recours à l'une et /ou à l'autre des dispositions suivantes :
- la partie remplaçable comprend au moins le récipient ;
- la partie à demeure comporte l'ensemble du mécanisme d'entraînement ;
- le mécanisme d'entraînement comprend une partie applicatrice au contact de l'organe, et adaptée, dans l'état monté, pour être reliée au mécanisme de transfert de mouvement pour appliquer ledit mouvement relatif de l'organe et du récipient selon ledit deuxième degré de liberté, et dans laquelle la partie remplaçable comprend au moins ladite partie applicatrice.

Les moteurs électriques positionnés sur le récipient, mais non sur l'équipage mobile, permettent de réaliser l'équipage mobile comme du consommable, sans avoir après chaque intervention à remplacer les moteurs électriques, qui sont parmi les éléments coûteux du système.

Selon un autre aspect, un enrouleur/dérouleur à cathéters comprend une base adaptée pour recevoir un organe tubulaire à introduire dans un canal d'un patient et s'étendant entre une première extrémité et une deuxième extrémité le long d'une direction d'élongation d'organe et un mécanisme d'entraînement adapté pour appliquer audit organe tubulaire au moins un mouvement le long de ladite direction d'élongation, ledit mécanisme d'entraînement comprenant :
- une plaquette adaptée pour porter l'organe et montée rotative par rapport à la base autour de ladite direction d'élongation,
- une partie applicatrice montée mobile sur la plaquette, au contact de l'organe,
- une vis sans fin, montée sur la plaquette et adaptée pour entraîner ladite partie applicatrice,
- un dispositif électrique de motorisation adapté pour entraîner la vis sans fin.

Un enrouleur/dérouleur selon une telle construction est relativement simple et peu coûteux à réaliser, tout en étant robuste et permettant une grande facilité d'utilisation.

D'autres caractéristiques et avantages de l'invention apparaîtront au cours de la description suivante de cinq de ses formes de réalisation, donnée à titre d'exemple non limitatif, en regard des dessins joints.

Sur les dessins :
- la figure 1 est une vue schématique en perspective d'un système d'artériographie,
- la figure 2 est une vue schématique en perspective d'un exemple de réalisation d'un enrouleur/dérouleur à cathéters,
- la figure 3a est une vue en perspective de dessus d'un mécanisme d'entraînement pour l'enrouleur/dérouleur de la figure 2,
- la figure 3b est une vue en perspective de dessous, du mécanisme d'entraînement de la figure 3a,
- la figure 4 est une vue partielle en perspective détaillée d'un exemple de réalisation d'un dispositif de fixation,
- la figure 5 est une vue en coupe détaillée d'un deuxième mode de réalisation,
- la figure 6 est une vue schématique de haut pour un troisième mode de réalisation,
- la figure 7 est une vue en coupe détaillée correspondant à la figure 5 pour un quatrième mode de réalisation,
- la figure 8 est une vue partielle en perspective de dessus d'une installation comprenant le mécanisme d'entraînement de la figure 3a, selon une variante de réalisation,
- la figure 9 est une vue partielle en perspective de dessus d'une installation comprenant le mécanisme d'entraînement de la figure 3a, selon une autre variante de réalisation, et
- la figure 10 est une vue partielle de dessus d'un cinquième mode de réalisation de l'invention.

Sur les différentes figures, les mêmes références désignent des éléments identiques ou similaires.

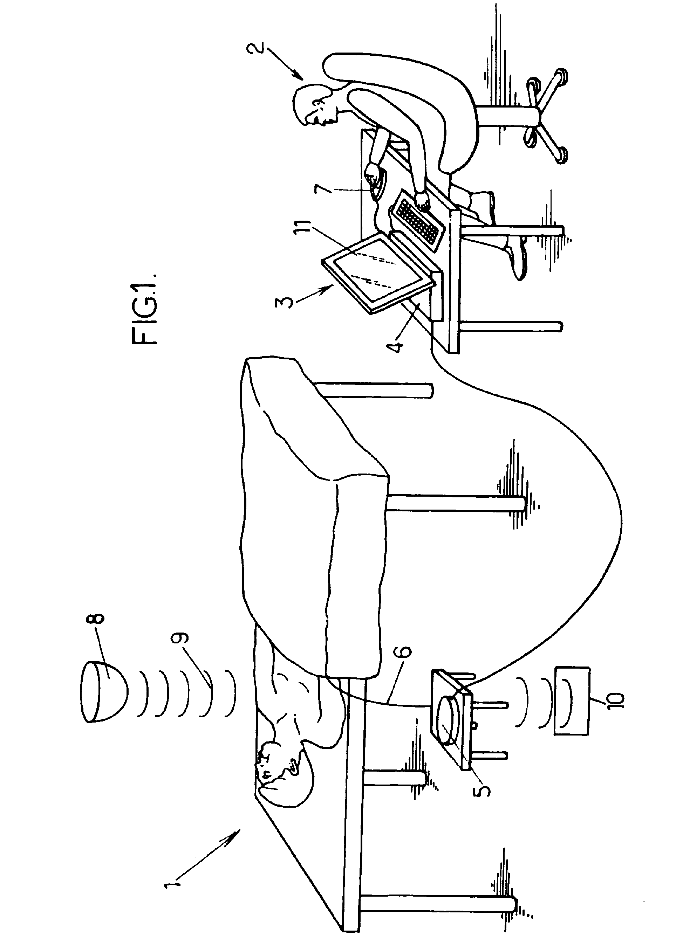

Sur la figure 1, un patient 1 est soumis à une artériographie. Cette artériographie est mise en oeuvre par un personnel adapté 2, tel que par exemple un chirurgien, ou du personnel médical qualifié, utilisant un système d'artériographie 3 automatisé qui comprend par exemple une machine programmable 4 commandant à distance un enrouleur/dérouleur 5 à cathéters disposé à proximité du patient 1.

Le déplacement d'un cathéter 6 à l'intérieur du corps du patient 1 est commandé à distance par le personnel qualifié 2 à l'aide d'un moyen de commande 7 tel que par exemple une souris, reliée à la machine programmable 4.

L'installation comprend en outre une source de rayons X 8 émettant des rayons X 9 vers le patient 1, et un détecteur de rayons X 10 apte à détecter le rayonnement traversant le patient 1. Le détecteur 10 peut être relié à l'ordinateur 4 pour afficher sur l'écran 11 de celui-ci une image détectée.

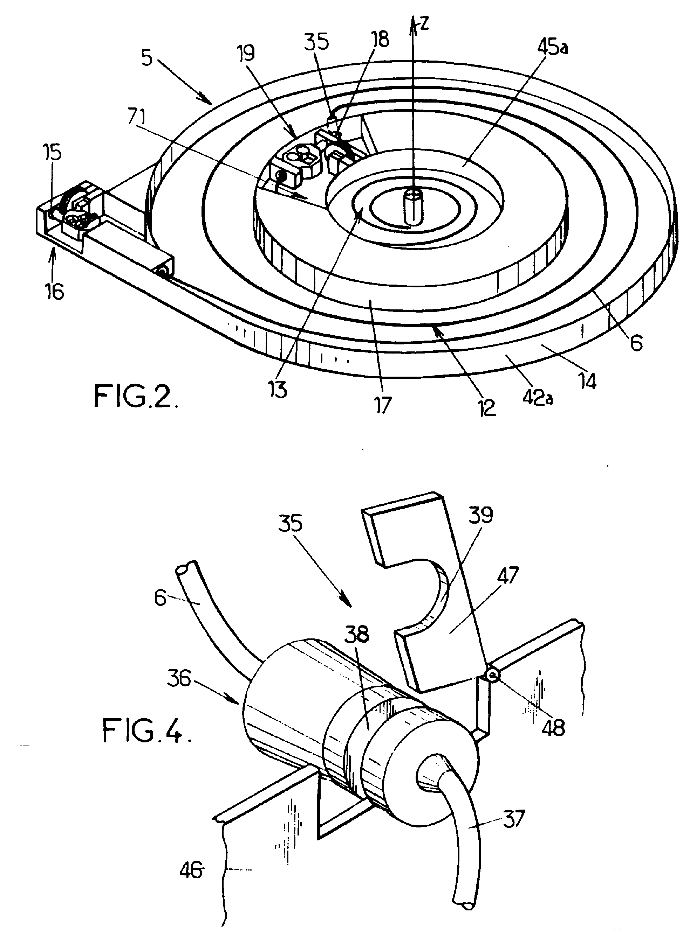

Comme représenté sur la figure 2, l'enrouleur/dérouleur 5 comporte au moins un premier système 12 et un deuxième système 13 concentriques disposés autour d'un axe par exemple orienté verticalement selon Z.

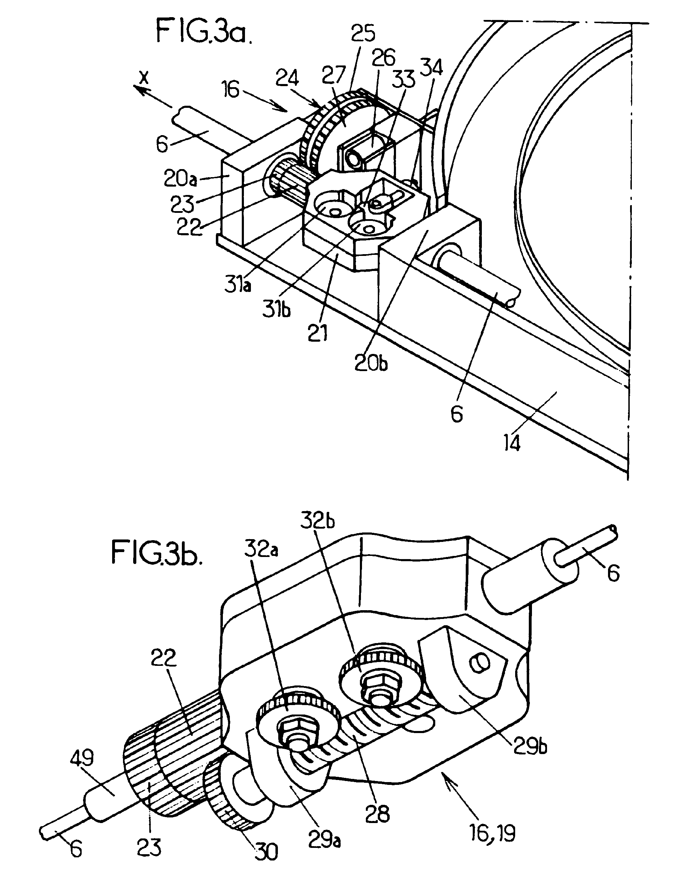

Le premier système 12 comporte un premier récipient 14 en forme de cuve creuse comportant une paroi externe 42a et une paroi interne 42b sensiblement verticales définissant entre elles un fond dans lequel est disposé, avant utilisation, un cathéter tubulaire destiné à être introduit dans une artère du patient 1. Ce cathéter s'étend d'une première extrémité à une deuxième extrémité le long d'une direction d'élongation de cathéter. La cuve 14 est par ailleurs remplie d'un liquide de conservation dans lequel baigne le cathéter. Le premier récipient 14 comporte une ouverture 15 à travers laquelle le cathéter peut atteindre le monde extérieur, et en particulier l'intérieur du corps du patient 1. Le premier système 12 comporte également un mécanisme d'entraînement 16 qui sera décrit plus en détail par la suite en relation avec les figures 3a et 3b. Ce mécanisme d'entraînement est par exemple situé en amont de l'ouverture 15 dans la direction d'élongation du cathéter, et est commandé par le chirurgien 2 par l'intermédiaire de l'ordinateur 4.

Le deuxième système 13 comporte un deuxième récipient 17 pouvant tourner autour de l'axe Z par rapport au premier récipient 14. A ce titre, on peut prévoir que le deuxième récipient 17 présente une ouverture centrale 45b coopérant avec la paroi interne 42b du premier récipient. Le deuxième récipient 17 comporte également une sortie 18 par laquelle un guide 37 contenu dans le deuxième récipient 17 peut accéder à l'extérieur du deuxième récipient, et en particulier à l'intérieur du premier récipient 14.

Le deuxième récipient 17 comprend une cuve à paroi externe 45a cylindrique qui reçoit un guide tubulaire s'étendant entre une première et une deuxième extrémité le long d'une direction d'élongation de guide, et baignant dans un liquide adapté à sa conservation. Par ailleurs, le deuxième récipient 17 comporte également un mécanisme d'entraînement 19, similaire au premier mécanisme d'entraînement 16 du premier système 12, disposé hors de la cuve, et qui sera décrit par la suite plus en détail en relation avec les figures 3a et 3b.

La figure 3a représente le premier mécanisme d'entraînement 16 selon un exemple illustratif de réalisation. Le deuxième mécanisme d'entraînement 19 pourra être réalisé de manière similaire. Le premier mécanisme d'entraînement 16 comporte un équipage mobile monté rotatif par rapport au premier récipient 14 autour de l'axe X d'élongation du cathéter 6 dans deux paliers 20a, 20b du premier récipient. L'équipage mobile comporte, dans l'exemple présenté, une plaquette 21 qui sera décrite plus en détail par la suite, portant le cathéter, et une bague d'engrenage de rotation 23 cylindrique montée sur un axe cylindrique 49, à l'intérieur desquels passe le cathéter 6.

Le premier mécanisme d'entraînement 16 comporte un moteur électrique de rotation 24 (non visible sur la figure 3a) entraînant en rotation, lors du passage d'un courant électrique, sur la commande provenant de l'ordinateur 4, la rotation d'un engrenage de rotation 25 qui coopère avec la bague d'engrenage de rotation 23 de l'équipage mobile.

Le mécanisme d'entraînement 16 comporte également un moteur électrique de translation 26 qui entraîne en rotation, sous l'effet du passage du courant électrique, sous commande de l'ordinateur 4, un engrenage de translation 27 qui coopère avec la bague d'engrenage de translation 22 montée rotative librement sur l'axe cylindrique 49 solidaire de la bague d'engrenage de rotation 23 à l'intérieur duquel passe le cathéter 6.

Dans une variante, on pourra prévoir de n'utiliser qu'un seul moteur couplé alternativement à l'un ou à l'autre des engrenages de rotation 25 et de translation 27. Ces moteurs sont par exemple des moteurs à pas.

Comme représenté sur la figure 3b, la bague d'engrenage de translation 22 entraîne en rotation une vis sans fin 28, montée rotative dans deux paliers 29a et 29b par l'intermédiaire d'un engrenage intermédiaire 30. La rotation autour d'un axe parallèle à l'axe X, de la vis sans fin 28 entraîne la rotation, autour de l'axe Z, de deux roues d'entraînement 31a et 31b disposées sur la face supérieure de la plaquette (figure 3a) par l'intermédiaire de roues dentées 32a et 32b correspondantes.

Le cathéter 6 est maintenu au contact de la tranche des roues d'entraînement 31 par une roulette de sollicitation 33, non motorisée, dont la position par rapport à la plaquette est définie par l'intermédiaire d'un mécanisme de réglage 34 permettant d'adapter le mécanisme d'entraînement 16 présentement décrit à différents diamètres de cathéters. Le cathéter étant maintenu en engagement avec les roues d'entraînement 31a et 31b par la roulette de sollicitation 33, la rotation de ces roues d'entraînement entraîne un déplacement du cathéter le long de son axe X d'élongation. Ces roues d'entraînement 31a, 31b forment donc une partie applicatrice, appliquant un mouvement au cathéter.

Comme représenté sur la figure 2, et plus dans le détail sur la figure 4, la sortie 18 du deuxième récipient 17 comporte un dispositif de fixation 35 permettant la libre rotation du cathéter 6 autour de son axe d'élongation par rapport au deuxième récipient 17, et l'entraînement du deuxième récipient 17 lors de la translation du cathéter 6 le long de sa direction d'élongation. Selon un exemple purement illustratif du dispositif de fixation 35 représenté sur la figure 4, l'extrémité distale du cathéter 6 est fixée sur une bague 36 présentant une rainure 38 qui s'insère dans un orifice 39 complémentaire formé dans la paroi 46 du deuxième récipient. Cet orifice 39 est formé pour moitié dans la paroi 46, et pour moitié dans un volet 47 articulé par exemple par une charnière 48 sur la paroi 46. La figure 4 représente d'ailleurs le volet 47 en position ouverte, pour l'insertion de la bague 36 dans l'orifice 39. La bague 36 est ainsi libre de tourner par rapport à la paroi 46 du deuxième récipient 17 autour d'un axe Y d'élongation du guide 37. La bague peut présenter une forme évasée vers le cathéter 6, pour faciliter sa préhension manuelle par un utilisateur.

Selon une variante, le volet pourrait être remplacé par un anneau ouvert rapporté sur la paroi.

On notera que le mode de réalisation présenté sur la figure 2 peut par exemple être réalisé modulaire, à savoir que le premier système 12 et le deuxième système 13 peuvent être commercialisés séparément et assemblés pour les besoins de l'examen du patient.

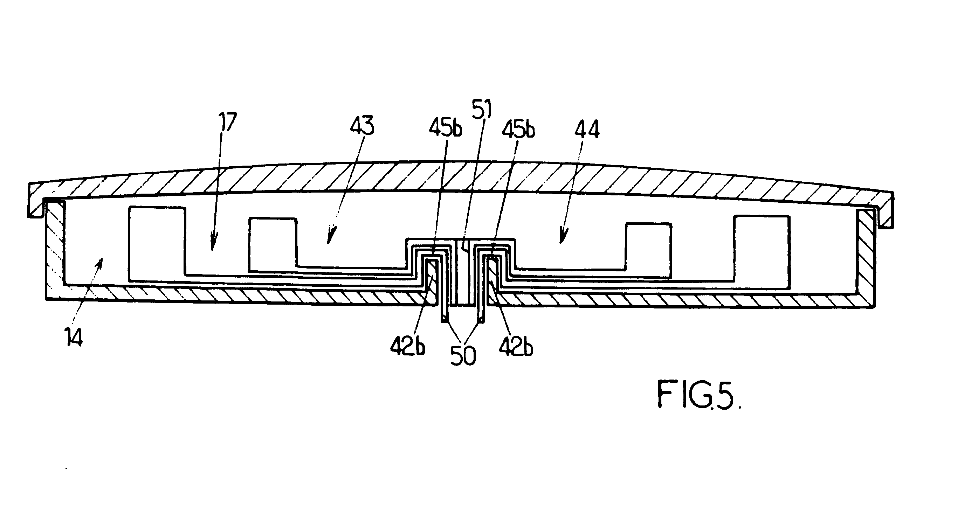

On peut d'ailleurs prévoir en outre, un troisième système 43 insérable dans le deuxième système et concentrique autour de l'axe Z avec celui-ci, pour l'insertion dans le patient d'un cathéter interventionel, en plus du guide et du cathéter comme représenté sur la figure 5. De manière similaire le troisième système comporte un récipient 44 comprenant une cuve pouvant contenir un liquide, un mécanisme d'entraînement et une sortie débouchant dans le deuxième système. Les systèmes sont disposés de sorte que l'élément à insérer présentant le plus large diamètre (généralement le cathéter) est placé dans le système externe, et l'élément à insérer présentant le plus fin diamètre (généralement le guide) est placé dans le système interne, le cathéter interventionel étant par conséquent placé dans le système intermédiaire.

En outre, chaque système peut lui-même être réalisé de manière modulaire, à savoir que les moteurs électriques peuvent être démontés du reste du système pour être remontés sur un enrouleur/dérouleur commercialisé, à titre de consommable, sans aucun moteur électrique. De cette manière, on diminue grandement les coûts liés à la réutilisation du système, car on peut garder d'une utilisation à l'autre les éléments les plus coûteux (ceux-ci pouvant éventuellement être stérilisés ou décontaminés avant leur utilisation ultérieure).

Un exemple d'utilisation du dispositif représenté sur les figures 1 et 2 est décrit ci-après. Un chirurgien ponctionne une artère, par exemple l'artère fémorale au niveau du pli inguinal et met en place un court tuyau comportant une valve réalisant une porte d'accès entre l'extérieur et l'artère, communément appelé désilé. L'enrouleur/dérouleur de la figure 2 est placé à proximité du patient, et est relié à l'ordinateur 4. L'enrouleur/dérouleur 5 contient déjà un liquide de conservation dans lequel baigne, dans le premier système, un cathéter, et dans le deuxième système un guide insérable dans le cathéter. En débrayant la roulette 33 du premier système, le cathéter peut être déplacé jusqu'à être inséré manuellement par le chirurgien à travers le désilé dans l'artère. Puis, depuis l'ordinateur 4, le chirurgien commande le moteur électrique de translation du deuxième système pour guider le guide 37, à travers la sortie 18, à l'intérieur du cathéter 6, jusqu'à ce que la première extrémité du guide arrive, à l'intérieur du patient, au niveau de la première extrémité du cathéter. Pendant cette opération, la source de rayons X 8 peut émettre des rayonnements qui n'ont pas d'influence sur le chirurgien 2, et l'image par le détecteur 10 peut être affichée sur l'écran 11 de l'ordinateur.

Pour que l'extrémité du cathéter atteigne l'emplacement d'intérêt à l'intérieur du corps du patient 1, le chirurgien 2 commande, depuis l'ordinateur 4, les fonctions suivantes :
- translation du guide : par activation du moteur électrique de translation 26 du deuxième système, qui entraîne en rotation la bague d'engrenage 22 de translation, l'engrenage intermédiaire 30, la vis sans fin 28, les roues dentées 32a et 32b et de ce fait, les roues d'entraînement 31a et 31b qui génèrent une translation du guide le long de sa direction d'élongation à l'intérieur du cathéter 6,
- la rotation du guide autour de l'axe longitudinal du guide, en commandant l'actionnement du moteur électrique de rotation 24 du deuxième système, et par là de l'engrenage de rotation 25 et de la bague d'engrenage de rotation 23 qui entraîne en rotation l'ensemble de l'équipage mobile et du guide par rapport aux paliers 20a et 20b du deuxième système, et par là celle du guide maintenu sur l'équipage par les roues d'entraînement 31a, 31b et la roulette 33,
- la translation du cathéter le long de sa direction d'élongation de cathéter par la commande similaire à la commande de translation du guide décrit précédemment, (du fait de la liaison de la deuxième extrémité du cathéter au dispositif de fixation 35, cette translation entraîne la rotation libre du deuxième système autour de l'axe Z par rapport au premier récipient), et
- la rotation du cathéter, commandée comme décrit précédemment pour le guide, le dispositif de fixation 35 autorisant le fait que le cathéter 6 tourne autour de son axe d'élongation sans influer sur le guide 37 ni le deuxième récipient 17.

On peut en outre prévoir que dans certains modes de réalisation, on peut commander conjointement la translation du guide et du cathéter, par une commande simultanée des deux moteurs correspondants.

Les commandes à la fois de translation et de rotation peuvent être bien entendu effectuées dans un sens ou dans l'autre, pour déplacer le guide et le cathéter jusqu'au site, où les en ramener.

Une rotation rapide du deuxième récipient par rapport au premier récipient peut être obtenue en entraînant en rotation l'ouverture centrale 45b du deuxième récipient par un moteur électrique (non représenté) spécialement dédié connecté sous le fond de la cuve à l'extrémité 50 correspondante de cette ouverture. Ce moteur dédié peut en particulier être utilisé pour le rembobinage du guide après utilisation.

A titre d'alternative, ce moteur dédié peut être remplacé pour la fonction de rembobinage par un ressort dédié bandé par le mouvement du récipient entraîné lors du déploiement du cathéter, qui est relié à son ouverture.

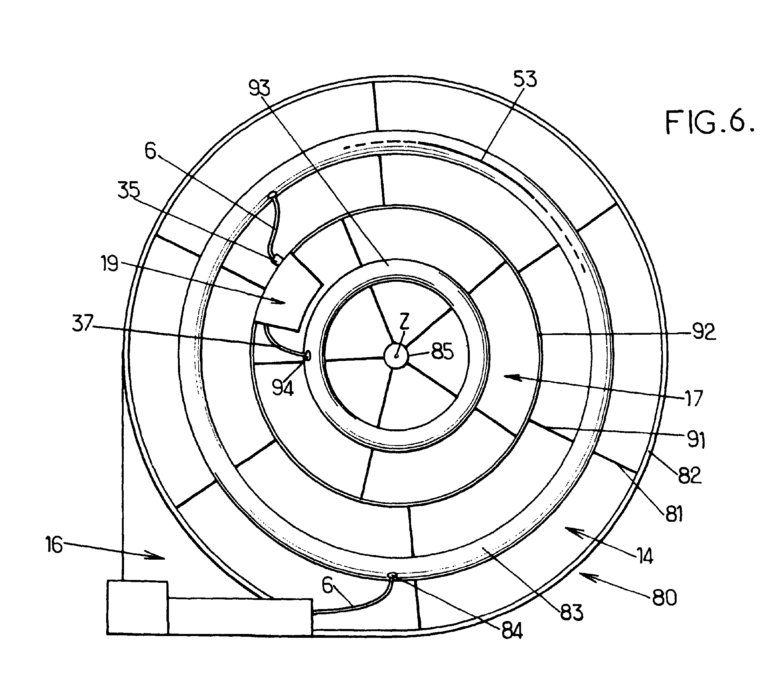

Comme représenté sur la figure 6, selon un deuxième mode de réalisation, on utilise, à titre de récipient du premier système, un premier plateau 80 comprenant une pluralité de rayons 81 s'étendant depuis un moyeu central 85 jusqu'à un bord périphérique 82. Ce plateau 80 porte un premier tuyau 83 recevant de manière interne le cathéter 6 baignant dans un liquide de conservation adapté. Le premier tuyau 83 comporte une ouverture 84 pour la sortie du cathéter vers le premier mécanisme d'entraînement 16 (non représenté à nouveau) identique à celui de la figure 2.

Sur le premier plateau 80 est posé un deuxième plateau 90 similaire au premier plateau, adapté pour tourner autour de l'axe Z par rapport au moyeu 85. Le deuxième plateau 90 comporte des rayons 91 s'étendant jusqu'à un bord périphérique 52, et porte un deuxième tuyau 93 recevant de manière interne le guide 37. Ce tuyau comporte une ouverture 94 pour la sortie du guide vers le deuxième mécanisme d'entraînement 19 (non représenté à nouveau). La sortie 35 est réalisée telle que décrit précédemment. Le guide 37 pénètre dans le premier tuyau 83 par l'intermédiaire d'une fente allongée 53 de celui-ci s'étendant par exemple sur la totalité de celui-ci (seulement partiellement représentée). En fonctionnement, la rotation du deuxième plateau 90 par rapport au moyeu entraîne la rotation du deuxième tuyau 93 porté par celui-ci.

En outre, dans un mode de réalisation comprenant également l'insertion d'un cathéter interventionnel, destiné à apporter une certaine fonction au site, les opérations et variantes décrites ci-dessus peuvent également être mises en place pour le troisième système.

A ce titre, l'extrémité 51 du troisième récipient peut également être entraînée par un moteur électrique dédié (non représenté) pour le rembobinage du guide dans un enrouleur/dérouleur à trois systèmes.

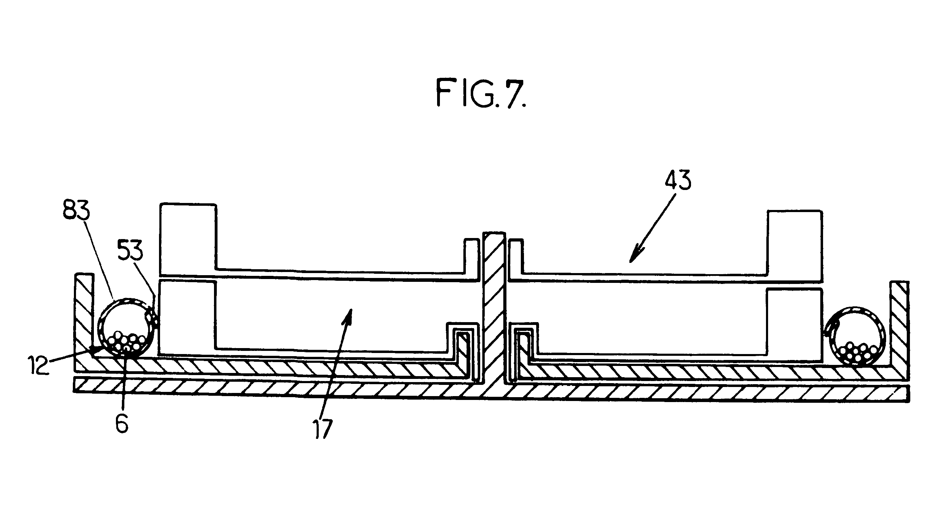

La figure 7 est une vue correspondant à la figure 5 pour un troisième mode de réalisation. Dans ce mode de réalisation, le troisième système 43 est superposé au deuxième système, et non plus imbriqué. Par conséquent, la sortie du troisième système n'est plus connectée directement en entrée du deuxième système, comme sur la Figure 5, mais peut être connectée directement sur l'entrée du premier système. La connexion au premier système du deuxième ou du troisième système peut avoir lieu de manière alternative. Par exemple, une fois le guide introduit dans le corps du patient, puis le cathéter le long du guide, le guide peut être retiré du corps du patient, et la bague 36 démontée du deuxième récipient et fixée au troisième récipient, contenant le cathéter interventionnel, pour introduction dans le corps du patient via le cathéter.

En variante, les deuxième et troisième systèmes peuvent être utilisés en parallèle, pour l'introduction en parallèle, non coaxiale, d'un guide et d'un cathéter interventionnel disposés respectivement dans les deuxième et troisième récipients. On peut alors prévoir, à l'extrémité arrière du cathéter contenu dans le premier récipient, une bague en T (non représentée) comportant une première entrée pour la connexion, avec liberté de rotation, au deuxième récipient et une deuxième entrée pour la connexion, avec liberté de rotation, au troisième récipient.

Comme représenté également sur la figure 7, le cathéter 6 est disposé dans le tuyau 83 étanche, par exemple en matière plastique, adapté pour contenir également le liquide de conservation du cathéter. Ce tuyau 83 présente également la fente 53 réalisée par exemple sous forme d'une lèvre mince, pour l'entrée du guide dans le tuyau.

L'utilisation de tuyaux n'est pas limitée au mode de réalisation des Figures 6 et 7, et est envisagée pour tout autre mode de réalisation de l'enrouleur/dérouleur.

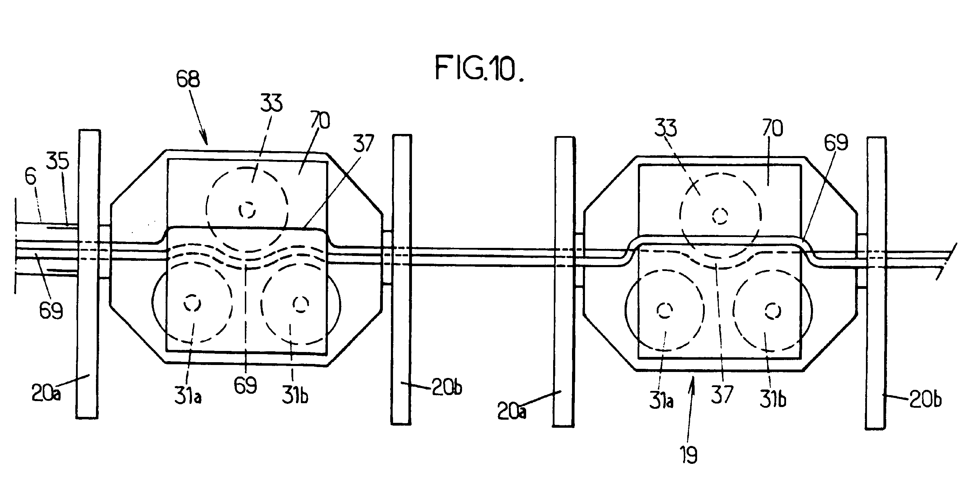

Selon un cinquième mode de réalisation, représenté sur la Figure 10, on fait passer le guide 37 et le cathéter interventionnel 69 en parallèle dans le cathéter 6 en utilisant seulement deux récipients.

Ce mode de réalisation correspond sensiblement à celui des figures 1 à 4 avec la fixation du cathéter 6 sur le deuxième récipient par l'intermédiaire du dispositif de fixation 35. Toutefois, les deuxième et troisième mécanismes d'entraînement 19 et 68 sont disposés à la suite dans l'emplacement 71 (Figure 2) ménagé à cet effet du deuxième système. Le guide et le cathéter interventionnel sont tous deux disposés dans le deuxième récipient. Comme représenté sur la figure 10, le deuxième mécanisme d'entraînement 19 entraîne le guide 37, le cathéter interventionnel 69 étant maintenu par une plaque 70 hors des roulettes 31a, 31b et 33. A la fois, le guide et le cathéter interventionnel passent dans les paliers 20a, 20b du deuxième mécanisme d'entraînement. De même, le cathéter interventionnel 69 est entraîné par le troisième mécanisme d'entraînement 68, le guide étant maintenu en dehors par une plaque 70. A la fois, le guide et le cathéter interventionnel passent dans les paliers 20a, 20b du troisième mécanisme d'entraînement. Ainsi, le deuxième mécanisme d'entraînement n'impartit aucun mouvement au cathéter interventionnel, et le troisième mécanisme d'entraînement n'impartit aucun mouvement au guide. Guide et cathéter interventionnel, tous deux débouchent en parallèle à l'intérieur du cathéter 6. Le cas échéant, le guide entre à l'intérieur du cathéter interventionnel par une ouverture dédiée, en aval du troisième mécanisme d'entraînement.

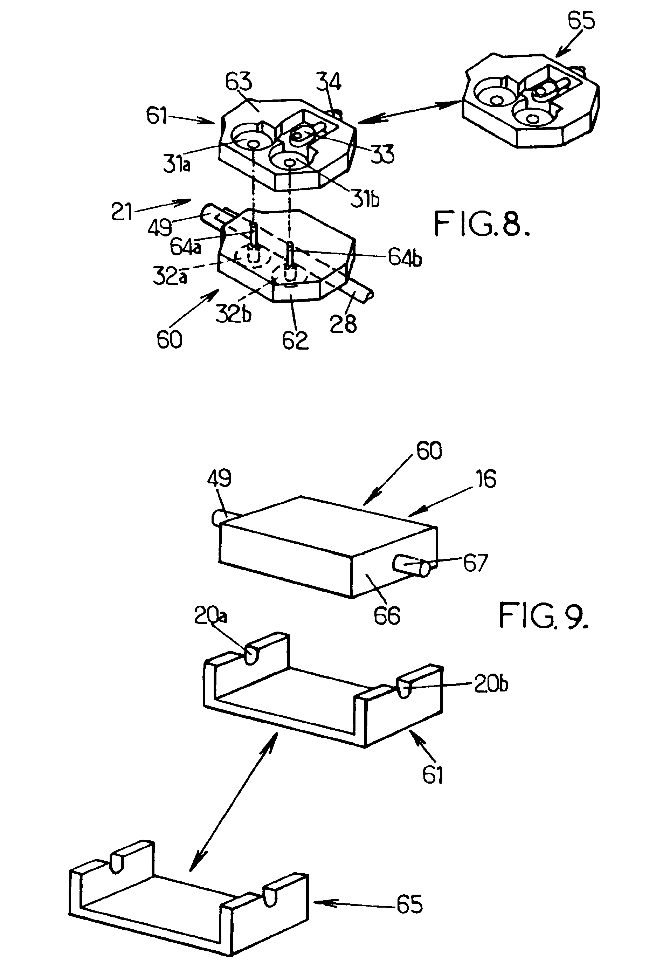

Comme représenté sur la Figure 8, l'installation peut comprendre une partie à demeure 60 et une partie remplaçable 61. Cette partie remplaçable 61 est une partie de l'enrouleur/dérouleur lui-même, et non pas les cathéters ou guides qui sont de toute façon classiquement échangés après chaque intervention. La partie remplaçable comprend par exemple tout ce qui a été souillé par le contact avec les guides ou cathéters, ou le liquide dans lequel ils baignent, au cours d'une intervention. La partie à demeure peut par exemple comprendre les moteurs électriques, qui constituent les parties coûteuses et difficilement recyclables de l'enrouleur/dérouleur. Deux exemples non limitatifs sont décrits par la suite en relation avec les figures 8 et 9.

Dans l'exemple de réalisation de la figure 8, la plaquette 21 est réalisée en deux parties :
- une partie inférieure 62 portant la vis sans fin 28, les roues dentées 32a, 32b et l'axe cylindrique 49, et
- une partie supérieure 63 portant les roulettes 31a, 31b, la roulette de sollicitation 33 et le mécanisme de réglage 34.

Dans un état assemblé, la partie remplaçable, formée par la partie supérieure de la plaquette, voit ses roulettes 31a, 31b enfichées sur les axes d'entraînement 64a, 64b liés aux roues dentées, respectivement.

Après utilisation de l'enrouleur/dérouleur pour une première intervention, la partie remplaçable 61 utilisée pour cette première intervention peut être retirée de la partie à demeure, et être remplacée par une partie de remplacement 65 identique à la partie remplaçable, pour une intervention ultérieure.

Dans le mode de réalisation de la figure 8, la partie supérieure 63 de la plaquette est réalisée sous forme de partie remplaçable, amovible par rapport à la partie à demeure. Alternativement, ou en combinaison, d'autres parties de l'installation peuvent être réalisées remplaçables.

Comme représenté sur la Figure 9, le mécanisme d'entraînement 16 tout entier est contenu dans un boîtier fermé 66 comportant l'axe 49 de sortie et un axe similaire 67 d'entrée. Le boîtier 66 forme la partie à demeure 60, et le récipient 14 forme la partie remplaçable montée amovible par rapport à la partie à demeure. Seule la partie du récipient servant à la réception du boîtier est représentée sur la Figure 9. Celle-ci comporte les deux paliers 20a, 20b, servant à la réception des axes 49 et 67, respectivement.

Après utilisation de l'enrouleur/dérouleur pour une première intervention, la partie remplaçable 61 peut être retirée de la partie à demeure, et être remplacée par une partie de remplacement 65 identique à la partie remplaçable, pour une intervention ultérieure. Par exemple, le récipient tout entier, éventuellement contenant l'organe, peut être remplacé pour chaque nouvelle intervention.

## Revendications

1. Enrouleur/dérouleur à cathéters comprenant au moins un premier système et un deuxième système,
le premier système comprenant :
- un premier récipient (14) adapté pour recevoir un premier organe tubulaire creux (6) allongé à introduire dans un canal d'un patient et s'étendant entre une première extrémité et une deuxième extrémité le long d'une direction d'élongation de premier organe,
ledit premier récipient comportant une ouverture (15) formée pour laisser passer ledit premier organe vers l'extérieur du premier système,
- un premier mécanisme d'entraînement (16) adapté pour appliquer au premier organe un mouvement à travers ladite ouverture par rapport audit premier récipient,
le deuxième système comprenant :
- un deuxième récipient (17) adapté pour recevoir un deuxième organe tubulaire (37) allongé à introduire dans le canal et s'étendant entre une première extrémité et une deuxième extrémité le long d'une direction d'élongation de deuxième organe,
ledit deuxième récipient comportant une sortie (18) formée pour laisser passer le deuxième organe vers l'extérieur du deuxième système et vers l'intérieur du premier système,
ledit deuxième récipient étant monté rotatif par rapport au premier récipient autour d'un axe de rotation (Z) ,
- un deuxième mécanisme d'entraînement (19) adapté pour appliquer au deuxième organe au moins un mouvement le long de ladite direction d'élongation de deuxième organe par rapport audit deuxième récipient,
ladite sortie comportant un dispositif de fixation (35) au premier organe, adapté pour un déplacement solidaire du premier organe et du deuxième récipient le long de la direction d'élongation de premier organe.

2. Enrouleur/dérouleur à cathéters selon la revendication 1 dans lequel le premier récipient présente un fond adapté pour recevoir ledit premier organe, et au moins une première surface de guidage (40 ; 42b),
dans lequel le deuxième récipient (17) présente un fond adapté pour recevoir ledit guide allongé, et au moins une deuxième surface de guidage (41 ; 45b) coopérant avec ladite première surface de guidage du premier récipient pour guider un mouvement relatif des premier et deuxième récipient autour dudit axe de rotation (Z).

3. Enrouleur/dérouleur à cathéters selon la revendication 1 ou la revendication 2 dans lequel le premier mécanisme d'entraînement (16) est adapté pour appliquer au premier organe au moins l'un des mouvements suivants par rapport audit premier récipient :
- une translation le long de ladite direction d'élongation de premier organe,
- une rotation autour de ladite direction d'élongation de premier organe.

4. Enrouleur/dérouleur à cathéters selon l'une des revendications précédentes dans lequel le deuxième mécanisme d'entraînement (19) est adapté pour appliquer au deuxième organe par rapport audit deuxième récipient
- une translation le long de ladite direction d'élongation de deuxième organe,
- une rotation autour de ladite direction d'élongation de deuxième organe.

5. Enrouleur/dérouleur à cathéters selon l'une des revendications précédentes, comprenant en outre un mécanisme de motorisation adapté pour impartir un mouvement relatif des premier (14) et deuxième (17) récipients autour dudit axe de rotation (Z).

6. Enrouleur/dérouleur à cathéters selon l'une des revendications précédentes comprenant en outre, à titre de premier organe, un cathéter (6) allongé s'étendant dans le premier récipient entre une première extrémité et une deuxième extrémité le long d'une direction d'élongation du premier organe, ladite deuxième extrémité étant fixée audit dispositif de fixation (35), le cathéter allongé coopérant avec ledit premier mécanisme d'entraînement (16) pour que celui-ci lui applique un mouvement par rapport audit premier récipient.

7. Enrouleur/dérouleur à cathéters selon la revendication 6 comprenant en outre à titre de deuxième organe un guide (37) allongé s'étendant dans le deuxième récipient entre une première extrémité et une deuxième extrémité le long d'une direction d'élongation de deuxième organe, le guide allongé coopérant avec ledit deuxième mécanisme d'entraînement pour que celui-ci lui applique un mouvement par rapport audit deuxième récipient,
la première extrémité du guide passant à travers ladite sortie (18) à l'intérieur du cathéter (6).

8. Enrouleur/dérouleur à cathéters selon l'une des revendications précédentes dans lequel les récipients (14, 17) contiennent un liquide adapté à la conservation d'organes à introduire dans un canal d'un patient.

9. Enrouleur/dérouleur à cathéters selon l'une des revendications précédentes dans lequel lesdits mécanismes d'entraînement (16, 19) sont réalisés amovibles par rapport à leur système respectif.

10. Enrouleur/dérouleur selon l'une quelconque des revendications précédentes dans lequel le deuxième récipient (17) est adapté pour recevoir en outre un troisième organe tubulaire (69) allongé à introduire dans le canal et s'étendant entre une première extrémité et une deuxième extrémité le long d'une direction d'élongation de troisième organe, le deuxième système comprenant un troisième mécanisme d'entraînement adapté pour appliquer au troisième organe au moins un mouvement le long de ladite direction d'élongation de troisième organe par rapport audit deuxième récipient, sans appliquer de mouvement au deuxième organe, le deuxième mécanisme d'entraînement étant adapté pour appliquer au deuxième organe ledit mouvement le long de la direction d'élongation de deuxième organe sans appliquer de mouvement au troisième organe.

11. Enrouleur/dérouleur à cathéters selon l'une des revendications 1 à 9 comprenant en outre un troisième système comprenant :
- un troisième récipient adapté pour recevoir un troisième organe tubulaire allongé à introduire dans le canal et s'étendant entre une première extrémité et une deuxième extrémité le long d'une direction d'élongation de troisième organe,
ledit troisième récipient comportant une sortie formée pour laisser passer le troisième organe,
ledit troisième récipient étant monté rotatif par rapport au deuxième récipient autour dudit axe de rotation,
- un troisième mécanisme d'entraînement adapté pour appliquer au troisième organe au moins un mouvement à travers ladite sortie du troisième récipient le long de ladite direction d'élongation de troisième organe par rapport audit troisième récipient,
la sortie du troisième récipient comportant un dispositif de fixation au deuxième organe, adapté pour un déplacement solidaire du deuxième organe et du troisième récipient le long de la direction d'élongation de deuxième organe.

12. Enrouleur/dérouleur à cathéters selon l'une des revendications 1 à 9 comprenant en outre un troisième système (43) comprenant :
- un troisième récipient (44) adapté pour recevoir un troisième organe tubulaire allongé à introduire dans le canal et s'étendant entre une première extrémité et une deuxième extrémité le long d'une direction d'élongation de troisième organe,
ledit troisième récipient comportant une sortie formée pour laisser passer le troisième organe,
ledit troisième récipient étant monté rotatif par rapport au premier récipient (14) autour dudit axe de rotation,
- un troisième mécanisme d'entraînement adapté pour appliquer au troisième organe au moins un mouvement à travers ladite sortie du troisième récipient le long de ladite direction d'élongation de troisième organe par rapport audit troisième récipient,
la sortie du troisième récipient comportant un dispositif de fixation au premier organe, adapté pour un déplacement solidaire du premier organe et du troisième récipient le long de la direction d'élongation de premier organe.

13. Enrouleur/dérouleur à cathéters selon l'une des revendications précédentes dans lequel au moins un mécanisme d'entraînement (16, 19) comprend un dispositif électrique de motorisation (24, 26) comprenant au moins un moteur électrique adapté pour générer, lors du passage d'un courant électrique, un mouvement relatif du récipient et d'un élément à déplacer correspondant choisi parmi lesdits organes.

14. Enrouleur/dérouleur à cathéters selon l'une quelconque des revendications précédentes, dans lequel chaque récipient (14, 17, 44) est en forme de cuve.

15. Installation comprenant un enrouleur/dérouleur à cathéters selon la revendication 1 comprenant un récipient (14, 17) adapté pour recevoir un organe (6, 37) tubulaire allongé à introduire dans un canal d'un patient et s'étendant entre une première extrémité et une deuxième extrémité le long d'une direction d'élongation,
le récipient comprenant une ouverture formée pour laisser passer ledit organe vers l'extérieur,
l'enrouleur/dérouleur comprenant en outre un mécanisme d'entraînement (16, 19) adapté pour appliquer à l'organe (6, 37) un mouvement à travers ladite ouverture par rapport audit récipient, ledit mécanisme d'entraînement comprenant :
- un équipage (21) monté mobile par rapport au récipient selon un premier degré de liberté, l'équipage portant l'organe,
- un dispositif électrique de motorisation adapté pour générer un mouvement lors du passage d'un courant électrique,
ledit dispositif électrique de motorisation étant adapté pour générer un mouvement relatif de l'équipage (21) et du récipient (14, 17) selon le premier degré de liberté, afin de générer un mouvement relatif de l'organe et du récipient selon ledit premier degré de liberté,
l'organe étant monté mobile par rapport à l'équipage (21) selon un deuxième degré de liberté distinct du premier degré de liberté,
ledit dispositif électrique de motorisation étant adapté pour générer un mouvement relatif de l'équipage et de l'organe selon le deuxième degré de liberté, afin de générer un mouvement relatif de l'organe et du récipient selon ledit deuxième degré de liberté,
ledit dispositif électrique de motorisation comprenant au moins un moteur électrique (24, 26), ledit dispositif électrique de motorisation comprenant au moins un élément de transfert de mouvement (23, 22, 30, 28, 32a, 32b) relié à un desdits moteurs électriques pour transmettre ledit mouvement relatif de l'organe et de l'équipage selon le deuxième degré de liberté depuis ledit moteur électrique,
l'installation comportant une partie à demeure (60), comportant lesdits moteurs électriques (24, 26), et une partie remplaçable utilisée (61), distincte dudit organe, montée de manière amovible par rapport à la partie à demeure, la partie à demeure et la partie remplaçable formant, dans un état monté, ledit enrouleur/dérouleur, l'installation comportant une pluralité de parties remplaçables de remplacement (65), identiques à la partie remplaçable utilisée (61).

16. Installation selon la revendication 15, dans laquelle la partie remplaçable comprend au moins le récipient (14, 17).

17. Installation selon la revendication 15 ou16, dans laquelle la partie à demeure (60) comporte l'ensemble du mécanisme d'entraînement (16, 19).

18. Installation selon la revendication 15 ou 16, dans laquelle le mécanisme d'entraînement (16, 19) comprend une partie applicatrice (31a, 31b, 33) au contact de l'organe (6, 37), et adaptée, dans l'état monté, pour être reliée au mécanisme de transfert de mouvement pour appliquer ledit mouvement relatif de l'organe et du récipient selon ledit deuxième degré de liberté, et dans laquelle la partie remplaçable (61) comprend au moins ladite partie applicatrice.

## Claims

1. A catheter winder/unwinder comprising at least a first system and a second system,
the first system comprising:
· a first receptacle (14) adapted to receive a first elongate tubular hollow member (6) for inserting into a duct of a patient and extending between a first end and a second end along a longitudinal direction of the first member;
said first receptacle including an opening (15) formed to allow said first member to pass towards the outside of the first system;
· a first drive mechanism (16) adapted to apply movement to the first member through said opening relative to said first receptacle;
the second system comprising:
· a second receptacle (17) adapted to receive a second elongate tubular member (37) for inserting into the duct and extending between a first end and a second end along a longitudinal direction of the second member;
said second receptacle having an outlet (18) formed to allow the second member to pass to the outside of the second system and towards the inside of the first system;
said second receptacle being mounted to rotate relative to the first receptacle about an axis of rotation (Z);
· a second drive mechanism (19) adapted to apply to the second member at least movement along said longitudinal direction of the second member relative to said second receptacle;
said outlet including a fastener device (35) for fastening to the first member and adapted to enable the first member and the second receptacle to move together along the longitudinal direction of the first member.

2. A catheter winder/unwinder according to claim 1,
wherein the first receptacle presents a bottom adapted to receive said first member, and at least a first guide surface (40 ; 42b);
wherein the second receptacle (17) presents a bottom adapted to receive said elongate guide, and at least a second guide surface (41, 45b) co-operating with said first guide surface of the first receptacle to guide the relative movement of the first and second receptacles about said axis of rotation (Z).

3. A catheter winder/unwinder according to claim 1 or 2, wherein the first drive mechanism (16) is adapted to apply to the first member at least one of the following kinds of movement relative to said first receptacle:
· movement in translation along said longitudinal direction of the first member; and
· movement in rotation about said longitudinal direction of the first member.

4. A catheter winder/unwinder according to any of claims 1 to 3, wherein the second drive mechanism (19) is adapted to apply to the second member relative to said second receptacle at least one of the following kinds of movement :
· movement in translation along said longitudinal direction of the second member; and/or
· movement in rotation about said longitudinal direction of the second member.

5. A catheter winder/unwinder according to any of claims 1 to 4, further including a motor mechanism adapted to impart relative movement of the first (14) and second (17) receptacles about said axis of rotation (Z).

6. A catheter winder/unwinder according to any of claims 1 to 5, further including, as its first member, an elongate catheter (6) extending in the first receptacle between a first end and a second end along a longitudinal direction of the first member, said second end being fastened to said fastener device (35), the elongate catheter co-operating with said first drive mechanism (16) so that it imparts movement thereto relative to said first receptacle.

7. A catheter winder/unwinder according to claim 6, further including, as its second member, an elongate guide (37) extending in the second receptacle between a first end and a second end along a longitudinal direction of the second member, the elongate guide co-operating with said second drive mechanism so that it imparts movement thereto relative to said second receptacle;
the first end of the guide passing through said outlet (18) to the inside of the catheter (6).

8. A catheter winder/unwinder according to any of claims 1 to 7, wherein the receptacles (14, 17) contain a liquid suitable for preserving members for inserting in a duct in a patient.

9. A catheter winder/unwinder according to any of claims 1 to 8, wherein said drive mechanisms (16, 19) are made to be removable relative to their respective systems.

10. A catheter winder/unwinder according to any of claims 1 to 9, wherein the second receptacle (17) is adapted also to receive a third elongate tubular member (69) for insertion into the duct and extending between a first end and a second end along a longitudinal direction of the third member, the second system including a third drive mechanism adapted to apply to the third member at least movement along said longitudinal direction of the third member relative to said second receptacle without applying movement to the second member, the second drive mechanism being adapted to apply to the second member said movement along the longitudinal direction of the second member without applying movement to the third member.

11. A catheter winder/unwinder according to any of claims 1 to 9, further comprising a third system comprising:
· a third receptacle adapted to receive a third elongate tubular member for inserting in the duct and extending between a first end and a second end along a longitudinal direction of the third member;
said third receptacle including an outlet formed to allow the third member to pass therethrough;
said third receptacle being mounted to rotate relative to the second receptacle about said axis of rotation;
· a third drive mechanism adapted to apply to the third member at least movement through said outlet of the third receptacle along said longitudinal direction of the third member relative to said third receptacle;
the outlet of the third receptacle including a fastener device for fastening to the second member and adapted for the second member and the third receptacle to move together along the longitudinal direction of the second member.

12. A catheter winder/unwinder according to any of claims 1 to 9, further comprising a third system (43) comprising:
· a third receptacle (44) adapted to receive a third elongate tubular member for inserting in the duct and extending between a first end and a second end along a longitudinal direction of the third member;
said third receptacle including an outlet formed to allow the third member to pass therethrough;
said third receptacle being mounted to rotate relative to the first receptacle (14) about said axis of rotation;
· a third drive mechanism adapted to apply to the third member at least movement through said outlet of the third receptacle along said longitudinal direction of the third member relative to said third receptacle;
the outlet of the third receptacle including a fastener device for fastening to the first member and adapted to enable the first member and the third receptacle to move together along the longitudinal direction of the first member.

13. A catheter winder/unwinder according to any of claims 1 to 12, wherein at least one drive mechanism (16, 19) comprises an electric motoring device (24, 26) having at least one electric motor adapted, on being powered electrically, to generate relative movement of the receptacle and a corresponding element to be moved selected amongst said members.

14. A catheter winder/unwinder according to any preceding claim, wherein each receptacle (14, 17, 44) is in the form of a tank.

15. An installation including a catheter winder/unwinder according to claim 1 comprising a receptacle (14, 17) adapted to receive an elongate tubular member (6, 37) for insertion into a duct of a patient and extending between a first end and a second end along a longitudinal direction;
the receptacle having an opening formed to allow said member to pass to the outside;
the winder/unwinder further comprising a drive mechanism (16, 19) adapted to apply to the member (6, 37) movement through said opening relative to said receptacle, said drive mechanism comprising:
· an equipment (21) mounted to move relative to the receptacle in a first degree of freedom, the equipment carrying the member; and
· an electric motoring device adapted to generate movement when powered electrically;
said electric motoring device being adapted to generate relative movement between the equipment (21) and the receptacle (14, 17) in the first degree of freedom so as to generate relative movement of the member and of the receptacle in said first degree of freedom;
the member being mounted to move relative to the equipment (21) in a second degree of freedom distinct from the first degree of freedom;
said electric motoring device being adapted to generate relative movement of the equipment and of the member in the second degree of freedom in order to generate relative movement of the member and of the receptacle in said second degree of freedom;
said electric motoring device comprising at least an electric motor (24, 26), said electric motoring device comprising at least one movement transfer element (23, 22, 30, 28, 32a, 32b) connected to one of said electric motors to transmit said relative movement of the member and the equipment in the second degree of freedom from said electric motor;
the installation comprising a permanent portion (60) comprising said electric motors (24, 26), and a utilized replaceable portion (61), distinct from said member, mounted removably relative to the permanent portion, the permanent portion and the replaceable portion in an assembled state together forming said winder/unwinder, the installation having a plurality of replaceable replacement portions (65) that are identical to the utilized replacement portion (61).

16. An installation according to claim 15, wherein the replaceable portion comprises at least the receptacle (14, 17).

17. An installation according to claim 15, wherein the permanent portion (60) includes the entire drive mechanism (16, 19).

18. An installation according to claim 15, wherein the drive mechanism (16, 19) includes an application portion (31a, 31b, 33) in contact with the member (6, 37) and adapted, in the assembled state, to be connected to the movement transfer mechanism to apply said relative movement of the member and of the receptacle in said second degree of freedom, and wherein the replaceable portion (61) comprises at least said application portion.

## Patentansprüche

1. Katheter-Aufroller/Abroller, der mindestens ein erstes System und ein zweites System umfasst,
wobei das erste System aufweist:
- einen ersten Behälter (14), der geeignet ist, um ein erstes längliches, hohles, rohrförmiges Element (6) aufzunehmen, das in einen Kanal eines Patienten einzuführen ist und sich zwischen einem ersten Ende und einem zweiten Ende entlang einer Ausdehnungsrichtung des ersten Elements erstreckt,
wobei der erste Behälter eine Öffnung (15) aufweist, die geformt ist, um das erste Element nach außerhalb des ersten Systems durchzulassen,
- einen ersten Antriebsmechanismus (16), der geeignet ist, um dem ersten Element eine Bewegung durch die Öffnung hindurch bezüglich des ersten Behälters aufzuprägen,
wobei das zweite System aufweist:
- einen zweiten Behälter (17), der geeignet ist, um ein zweites längliches, rohrförmiges Element (37) aufzunehmen, das in den Kanal einzuführen ist und sich zwischen einem ersten Ende und einem zweiten Ende entlang einer Ausdehnungsrichtung des zweiten Elements erstreckt,
wobei der zweite Behälter einen Ausgang (18) aufweist, der geformt ist, um das zweite Element nach außerhalb des zweiten Systems und ins Innere des ersten Systems durchzulassen,
wobei der zweite Behälter bezüglich des ersten Behälters um eine Drehachse (Z) drehbar montiert ist,
- einen zweiten Antriebsmechanismus (19), der geeignet ist, um dem zweiten Element mindestens eine Bewegung entlang der Ausdehnungsrichtung des zweiten Elements bezüglich des zweiten Behälters aufzuprägen,
wobei der Ausgang eine Vorrichtung (35) zur Befestigung am ersten Element aufweist, die für eine gemeinsame Verschiebung des ersten Elements und des zweiten Behälters entlang der Ausdehnungsrichtung des ersten Elements geeignet ist.

2. Katheter-Aufroller/Abroller nach Anspruch 1, bei dem der erste Behälter einen Boden, der geeignet ist, um das erste Element aufzunehmen, und mindestens eine erste Führungsfläche (40; 42b) aufweist,
bei dem der zweite Behälter (17) einen Boden, der geeignet ist, um die längliche Führung aufzunehmen, und mindestens eine zweite Führungsfläche (41; 45b) aufweist, die mit der ersten Führungsfläche des ersten Behälters zusammenwirkt, um eine relative Bewegung des ersten und des zweiten Behälters um die Drehachse (Z) zu führen.

3. Katheter-Aufroller/Abroller nach Anspruch 1 oder Anspruch 2, bei dem der erste Antriebsmechanismus (16) geeignet ist, um dem ersten Organ mindestens eine der folgenden Bewegungen bezüglich des ersten Behälters aufzuprägen:
- eine Translationsbewegung entlang der Ausdehnungsrichtung des ersten Elements,
- eine Drehung um die Ausdehnungsrichtung des ersten Elements.

4. Katheter-Aufroller/Abroller nach einem der vorhergehenden Ansprüche, bei dem der zweite Antriebsmechanismus (19) geeignet ist, um dem zweiten Element bezüglich des zweiten Behälters aufzuprägen:
- eine Translationsbewegung entlang der Ausdehnungsrichtung des zweiten Elements,
- eine Drehung um die Ausdehnungsrichtung des zweiten Elements.

5. Katheter-Aufroller/Abroller nach einem der vorhergehenden Ansprüche, der weiter einen Motorisierungsmechanismus aufweist, der geeignet ist, um eine relative Bewegung des ersten (14) und des zweiten (17) Behälters um die Drehachse (Z) zu ermöglichen.

6. Katheter-Aufroller/Abroller nach einem der vorhergehenden Ansprüche, der weiter als erstes Element einen länglichen Katheter (6) aufweist, der sich im ersten Behälter zwischen einem ersten Ende und einem zweiten Ende entlang einer Ausdehnungsrichtung des ersten Elements erstreckt, wobei das zweite Ende an der Befestigungsvorrichtung (35) befestigt ist, wobei der längliche Katheter mit dem ersten Antriebsmechanismus (16) zusammenwirkt, damit dieser ihm eine Bewegung bezüglich des ersten Behälters aufprägt.

7. Katheter-Aufroller/Abroller nach Anspruch 6, der weiter als zweites Element eine längliche Führung (37) aufweist, die sich in dem zweiten Behälter zwischen einem ersten Ende und einem zweiten Ende entlang einer Ausdehnungsrichtung des zweiten Elements erstreckt, wobei die längliche Führung mit dem zweiten Antriebsmechanismus zusammenwirkt, damit dieser ihr eine Bewegung bezüglich des zweiten Behälters aufprägt,
wobei das erste Ende der Führung durch den Ausgang (18) ins Innere des Katheters (6) geht.

8. Katheter-Aufroller/Abroller nach einem der vorhergehenden Ansprüche, bei dem die Behälter (14, 17) eine Flüssigkeit enthalten, die für die Konservierung von Elementen geeignet ist, die in einen Kanal eines Patienten einzuführen sind.

9. Katheter-Aufroller/Abroller nach einem der vorhergehenden Ansprüche, bei dem die Antriebsmechanismen (16, 19) bezüglich ihres jeweiligen Systems entfernbar hergestellt sind.

10. Aufroller/Abroller nach einem der vorhergehenden Ansprüche, bei dem der zweite Behälter (17) geeignet ist, um außerdem ein drittes längliches rohrförmiges Element (69) aufzunehmen, das in den Kanal einzuführen ist und sich zwischen einem ersten Ende und einem zweiten Ende entlang einer Ausdehnungsrichtung des dritten Elements erstreckt, wobei das zweite System einen dritten Antriebsmechanismus aufweist, der geeignet ist, um dem dritten Element mindestens eine Bewegung entlang der Ausdehnungsrichtung des dritten Elements bezüglich des zweiten Behälters aufzuprägen, ohne dem zweiten Element eine Bewegung aufzuprägen, wobei der zweite Antriebsmechanismus geeignet ist, um dem zweiten Element die Bewegung entlang der Ausdehnungsrichtung des zweiten Elements aufzuprägen, ohne dem dritten Element eine Bewegung aufzuprägen.

11. Katheter-Aufroller/Abroller nach einem der Ansprüche 1 bis 9, der weiter ein drittes System umfasst, das aufweist:
- einen dritten Behälter, der geeignet ist, um ein drittes längliches, rohrförmiges Element aufzunehmen, das in den Kanal einzuführen ist und sich zwischen einem ersten Ende und einem zweiten Ende entlang einer Ausdehnungsrichtung des dritten Elements erstreckt,
wobei der dritte Behälter einen Ausgang aufweist, der geformt ist, um das dritte Element durchzulassen,
wobei der dritte Behälter bezüglich des zweiten Behälters um die Drehachse drehbar montiert ist,
- einen dritten Antriebsmechanismus, der geeignet ist, um dem dritten Element mindestens eine Bewegung bezüglich des dritten Behälters durch den Ausgang des dritten Behälters hindurch entlang der Ausdehnungsrichtung des dritten Elements aufzuprägen,
wobei der Ausgang des dritten Behälters eine Vorrichtung zur Befestigung an dem zweiten Element aufweist, die für eine gemeinsame Verschiebung des zweiten Elements und des dritten Behälters entlang der Ausdehnungsrichtung des zweiten Elements geeignet ist.

12. Katheter-Aufroller/Abroller nach einem der Ansprüche 1 bis 9, der außerdem ein drittes System (43) aufweist, das aufweist:
- einen dritten Behälter (44), der geeignet ist, um ein drittes längliches, rohrförmiges Element aufzunehmen, das in den Kanal einzuführen ist und sich zwischen einem ersten Ende und einem zweiten Ende entlang einer Ausdehnungsrichtung des dritten Elements erstreckt,
wobei der dritte Behälter einen Ausgang aufweist, der geformt ist, um das dritte Element durchzulassen,
wobei der dritte Behälter bezüglich des ersten Behälters (14) um die Drehachse drehbar montiert ist,
- einen dritten Antriebsmechanismus, der geeignet ist, um dem dritten Element mindestens eine Bewegung bezüglich des dritten Behälters durch den Ausgang des dritten Behälters hindurch entlang der Ausdehnungsrichtung des dritten Elements aufzuprägen,
wobei der Ausgang des dritten Behälters eine Vorrichtung zur Befestigung am ersten Element aufweist, die für eine gemeinsame Verschiebung des ersten Elements und des dritten Behälters entlang der Ausdehnungsrichtung des ersten Elements geeignet ist.

13. Katheter-Aufroller/Abroller nach einem der vorhergehenden Ansprüche, bei dem mindestens ein Antriebsmechanismus (16, 19) eine elektrische Motorisierungsvorrichtung (24, 26) aufweist, die mindestens einen Elektromotor aufweist, der geeignet ist, um beim Durchgang eines elektrischen Stroms eine relative Bewegung des Behälters und eines entsprechenden zu verschiebenden Bauteils zu erzeugen, das aus den Elementen ausgewählt wird.

14. Katheter-Aufroller/Abroller nach einem der vorhergehenden Ansprüche, bei dem jeder Behälter (14, 17, 44) die Form einer Wanne hat.

15. Anlage, die einen Katheter-Aufroller/Abroller nach Anspruch 1 aufweist, der einen Behälter (14, 17) aufweist, der geeignet ist, um ein längliches, rohrförmiges Element (6, 37) aufzunehmen, das in einen Kanal eines Patienten einzuführen ist und sich zwischen einem ersten Ende und einem zweiten Ende entlang einer Ausdehnungsrichtung erstreckt,
wobei der Behälter eine Öffnung aufweist, die geformt ist, um das Element nach außen durchzulassen,
wobei der Aufroller/Abroller außerdem einen Antriebsmechanismus (16, 19) aufweist, der geeignet ist, um dem Element bezüglich des Behälters (6, 37) eine Bewegung durch die Öffnung hindurch aufzuprägen, wobei der Antriebsmechanismus aufweist:
- eine Ausrüstung (21), die bezüglich des Behälters mit einem ersten Freiheitsgrad beweglich montiert ist, wobei die Ausrüstung das Element trägt,
- eine elektrische Motorisierungsvorrichtung, die geeignet ist, um beim Durchgang eines elektrischen Stroms eine Bewegung zu erzeugen,
wobei die elektrische Motorisierungsvorrichtung geeignet ist, um eine relative Bewegung der Ausrüstung (21) und des Behälters (14, 17) gemäß dem ersten Freiheitsgrad zu erzeugen, um eine relative Bewegung des Elements und des Behälters gemäß dem ersten Freiheitsgrad zu erzeugen,
wobei das Element bezüglich der Ausrüstung (21) mit einem zweiten Freiheitsgrad beweglich montiert ist, der sich von dem ersten Freiheitsgrad unterscheidet,
wobei die elektrische Motorisierungsvorrichtung geeignet ist, um eine relative Bewegung der Ausrüstung und des Elements gemäß dem zweiten Freiheitsgrad zu erzeugen, um eine relative Bewegung des Elements und des Behälters gemäß dem zweiten Freiheitsgrad zu erzeugen,
wobei die elektrische Motorisierungsvorrichtung mindestens einen Elektromotor (24, 26) aufweist,
wobei die elektrische Motorisierungsvorrichtung mindestens ein Bewegungsübertragungselement (23, 22, 30, 28, 32a, 32b) aufweist, das mit einem der Elektromotoren verbunden ist, um die relative Bewegung des Elements und der Ausrüstung gemäß dem zweiten Freiheitsgrad ausgehend von dem Elektromotor zu übertragen,
wobei die Anlage einen dauerhaft installierten Teil (60), der die Elektromotoren (24, 26) enthält, und einen genutzten austauschbaren Teil (61) enthält, der sich von dem Element unterscheidet und bezüglich des dauerhaft installierten Teils entfernbar montiert ist, wobei der dauerhaft installierte Teil und der entfernbare Teil in einem montierten Zustand den Aufroller/Abroller bilden, wobei die Anlage mehrere austauschbare Ersatzteile (65) aufweist, die gleich dem genutzten austauschbaren Teil (61) sind.

16. Anlage nach Anspruch 15, bei der der austauschbare Teil mindestens den Behälter (14, 17) enthält.

17. Anlage nach Anspruch 15 oder 16, bei der der dauerhaft installierte Teil (60) die Gesamtheit des Antriebsmechanismus (16, 19) enthält.

18. Anlage nach Anspruch 15 oder 16, bei der der Antriebsmechanismus (16, 19) ein Anwendungsteil (31a, 31b, 33) in Kontakt mit dem Element (6, 37) aufweist, das im montierten Zustand geeignet ist, um mit dem Bewegungsübertragungsmechanismus verbunden zu werden, um die relative Bewegung des Elements und des Behälters gemäß dem zweiten Freiheitsgrad aufzuprägen, und bei der der austauschbare Teil (61) mindestens das Anwendungsteil enthält.
